# EUROPÄISCHE PATENTSCHRIFT

(11) **EP 0 642 323 B1**
(45) Veröffentlichungstag und Bekanntmachung des Hinweises auf die Patenterteilung: **03.03.1999**
(21) Anmeldenummer: 94911063.9
(22) Anmeldetag: 19.03.1994
(51) Int. Cl.: A61B 17/58

(54) **FIXATIONSSTIFT ZUR OSTEOSYNTHESE**
FIXING PIN FOR OSTEOSYNTHESIS
TIGE DE FIXATION POUR OSTEOSYNTHESE

(30) Priorität: 25.03.1993 DE 4309707; 26.02.1994 DE 4406374
(43) Veröffentlichungstag der Anmeldung: 15.03.1995
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.
(86) Internationale Anmeldenummer: DE9400323
(87) Internationale Veröffentlichungsnummer: WO9421184

(56) Entgegenhaltungen:
- EP-A- 0 369 266
- EP-A- 0 424 734
- WO-A-93/08758
- FR-A- 2 499 400
- FR-A- 2 649 310

## Beschreibung

Die Erfindung bezieht sich auf einen Fixationsstift zur Durchführung von Osteosynthesearbeiten.

In der Osteosynthese ist es allgemein bekannt, Knochenfragmente durch Schrauben oder Nägel zu fixieren. Bei kleinen Knochenabsplitterungen sind die aber im Stand der Technik zur Verfügung stehenden Schrauben oder Nägel viel zu groß, um diese kleinen abgesplitterten Teilchen beschädigungslos am Hauptknochen festzulegen. Die Festlegung dieser Knochenfragmente mittels einfacher Stifte oder Nägel ist deshalb problematisch, da sich das Knochenteilchen auf der Außenwandung des Stiftes oder Nagels verschieben kann, da es nicht durch ein Widerlager gehalten wird.

Fixationsstifte sind durch die EP 314 021 A2 oder z. B. durch die DE-32 44 819 A1 bekannt, haben aber hier die Aufgabe, jeweils einen Fixateur extern an den Knochenfragmenten festzulegen. Bei diesen bekannten Fixationsstiften ist der Übergang vom Gewindeteil zum Schaftteil als geradlinige Stufe ausgebildet und die Festlegung der Knochenfragmente aneinander erfolgt nicht durch die Fixationsstifte, sondern durch den Fixateur extern, der über die Fixationsstifte festgelegt wird.

Außerdem beschreibt die FR-A-24 99 400 ebenfalls einen Fixateur externe, um Knochenfragmente festzulegen, der einen glattwandigen Schaftteil und einen Gewindeteil aufweist und wobei Schaftteil und Gewindeteil konisch ineinander übergehen. FR-A-2 649 310 beschreibt als bekannt Fixationsstifte für Osteosynthese arbeiten, die wenigstens teilweise mit Gewinde versehen sind, wobei der Stift abgeschitten werden kann

Würde man einen kleinen Fixationsstift zur Festlegung kleiner Knochenfragmente ebenfalls mit einer geradlinigen Stufe ausrüsten, würde beim Eindrehen des Fixationsstiftes diese Stufe an einer Umfangsstelle als erstes mit dem Knochenfragment in Berührung kommen und dadurch das Knochenfragment aufgrund der entstehenden Reibung verschieben.

Demgegenüber liegt der Erfindung die Aufgabe zugrunde, einen Fixationsstift für Knochenfragmente zu schaffen, der trotz seiner Kleinheit ein sicheres Festlegen des abgesplitterten Knochenfragmentes an dem Hauptknochen bewirkt.

Diese der Erfindung zugrundeliegende Aufgabe wird durch die Lehre des Hauptanspruches gelöst.

Mit anderen Worten ausgedrückt, wird ein Stift vorgeschlagen, der über einen großen Teil seiner Lange einen glattwandigen Schaftteil aufweist, wobei sich an das untere Ende dieses Schaftteils ein relativ kurzes und im Durchmesser geringeres Gewindeteil anschließt, wobei der Übergang vom Schaftteil zum Gewindeteil nicht als rechteckige Stufe ausgebildet ist, sondern konisch gestaltet ist, so daß beim Einschrauben ein geringfügiges Eindringen dieses konischen Übergangteiles in das abgesplitterte Knochenfragment möglich ist. Beim Einsatz dieses Stiftes wird das Gewindeteil durch das Knochenfragment in den Hauptknochenteil eingeschraubt, wobei sich nunmehr die Oberseite des Knochenfragmentes an die konische Übergangsstelle zwischen, Gewindeteil und Schaftteil anpreßt, d. h. einerseits wird das Knochenfragment nicht durch die Drehbewegung bewegt, andererseits kann sich das Knochenfragment nicht in Richtung auf das Schaftteil vom Hauptknochen ablösen.

Das Schafteil befindet sich im umgebenden Muskelgewebe und wird durch entsprechende Werkzeuge leicht hinsichtlich seiner Länge abgeschnitten, wobei das Schaftteil aus einem Material besteht, das ein solches Ablangen ermöglicht.

Der Gewindeteil des Fixationssstiftes ist mit Feingewinde ausgerüstet, so daß ein feinfühliges und genaues Eindrehen des Stiftes ermöglicht wird. Hierbei ist das Gewinde des Gewindeteiles als selbstschneidendes Gewinde ausgebildet

Weiterhin wird gemäß der Erfindung vorgeschlagen, daß auf den Fixationsstift eine Stützscheibe aufsetzbar ist, die sich großflächiger als die Stufe auf die Oberseite des zu fixierenden Knochens legen kann. Hierbei hat die Stützscheibe weiterhin die Aufgabe, ggf. mehrere Knochensplitterchen gleichzeitig festzulegen.

Die Stützscheibe weist dabei - vorzugsweise mittig - eine Bohrung auf, die einen Durchmesser aufweist, der größer als der Außendurchmesser des Gewindeteiles und kleiner als der Außendurchmesser des Schaftteiles ist, wobei die Bohrung weiterhin in ihrer Form der konischen Form der Stufe angepaßt ist.

Es ist aber auch möglich, daß die Stützscheibe im Inneren der Bohrung ein Gewinde aufweist, das mit dem Gewinde des Gewindeteiles des Fixationsstiftes kämmen kann, so daß dadurch die Stützscheibe auf das Gewindeteil des Fixationsstiftes aufgeschraubt werden kann.

Ausführungsbeispiele der Erfindung werden nachfolgend anhand der Zeichnungen erläutert. Die Zeichnungen zeigen dabei in
- Fig. 1: in größerem Maßstab einen Fixationsstift gemäß der Erfindung, in
- Fig. 2: ein Anwendungsbeispiel des Fixationsstiftes, in
- Fig. 3: einen Fixationsstift mit aufgesetzter Stützscheibe und in
- Fig. 4: die Stützscheibe ohne Fixationsstift.

In den Zeichnungen ist ein Fixationsstift 1 dargestellt, der im wesentlichen aus einem glattwandigen Schaftteil 2 besteht, an daß sich ein Gewindeteil 3 anschließt, wobei der Außendurchmesser dieses Gewindeteiles 3 kleiner als der Außendurchmesser des Schafteiles 2 ist. Zwischen dem Gewindeteil 3 und dem Schaftteil wird auf diese Weise eine konische Stufe 4 ausgebildet, die einerseits als Widerlager für das durch das Gewindeteil am Hauptknochen A festgelegte Knochenfragment B dient, andererseits aber in der Lage ist, geringfügig in das Knochenfragment B einzudringen.

Um eine Vorstellung über die Größenordnung zu bekommen, wird darauf hingewiesen, daß ein solcher Fixationsstift 1 beispielsweise eine Länge von 100 mm aufweisen kann, wobei das Gewindeteil 3 eine Länge von 15 mm und das Schaftteil 2 eine Länge von 85 mm aufweist. Der Durchmesser des Gewindeteils kann 1,6 mm betragen, wenn der Durchmesser des Schaftteiles 2, 2 mm beträgt oder der Durchmesser des Schaftteiles beträgt 1,6 mm, dann weist das Gewindeteil einen Durchmesser von 1,2 mm auf.

Natürlich sind auch größere Durchmesser des Schaftteiles und des Gewindeteiles möglich.

Das Gewinde des Gewindeteiles ist als Feingewinde ausgebildet, so daß auch kleine Knochensplitterchen damit festgelegt werden können. Das Gewinde ist dabei selbstschneidend gestaltet.

Das Schaftteil ist leicht durch eine entsprechende Zange abzuschneiden, so daß überstehende Bereiche leicht entfernt werden können.

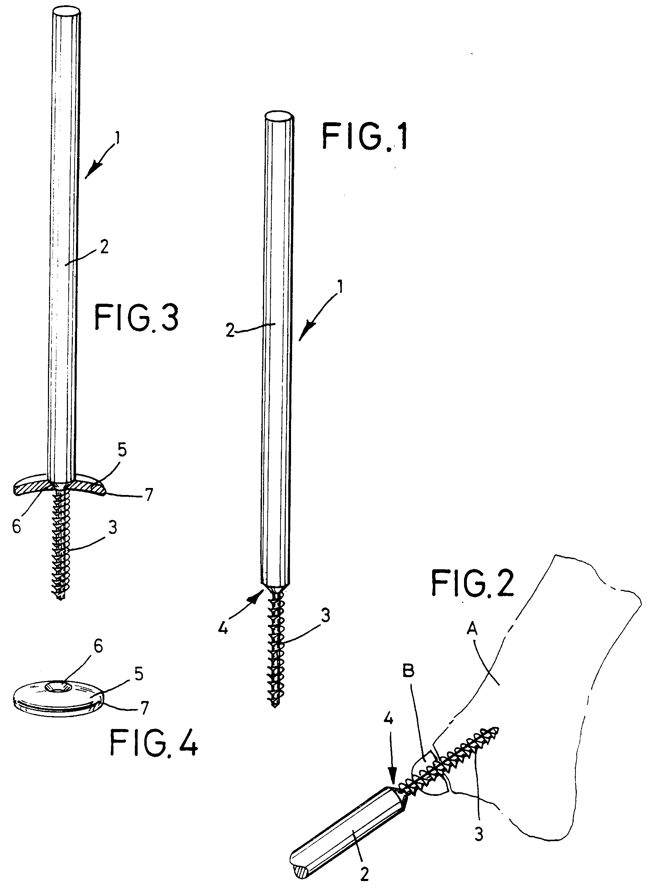

In Fig. 3 ist ein Fixationsstift 1 dargestellt, der eine Stützscheibe 5 trägt. Diese Stützscheibe 5 weint eine - vorzugsweise mittige - Bohrung 6 auf, durch die das Gewindeteil 3 des Fixationsstiftes 1 greifen kann, wobei sich dann die Stützscheibe 5 an der Stufe 4 des Fixationsstiftes 1 festlegt.

Um eine sichere und gute Festlegung zu erreichen, ist die Bohrung 6 in Anpassung an die konische Ausbildung der Stufe 4 ausgebildet, so daß sich die Stufe 4 satt in die Bohrung einlegt.

Die Stützscheibe 5 ist dabei leicht gewölbt ausgebildet und die konkave Seite ist zum festzulegenden Knochen hin gerichtet, wobei vorzugsweise auch die Randkanten 7 der Stützscheibe 5 abgerundet ausgebildet sind, so daß hier keine scharfkantigen und ggf. zu Verletzungen führenden Kanten vorhanden sind. Hierbei ist es möglich, Stützscheiben unterschiedlicher Größe und unterschiedlicher Wölbung vorzuhalten.

Während bei dem in Fig. 3 und 4 dargestellten Ausführungsbeispiel die Stützscheibe 5 frei auf dem Gewindeteil gleiten kann und sich nur über eine entsprechende konische Ausnehmung an die konische Stützstufe anpaßt, ist es auch in der Praxis möglich, daß die Stützscheibe ein dem Außengewinde des Gewindeteiles des Fixationsstiftes angepaßtes Innengewinde im Bereich der Bohrung aufweist, so daß dadurch dann die Stützscheibe auf das Gewindeteil des Fixationsstiftes aufgeschraubt werden kann.

## Patentansprüche

1. Fixationsstift (1) für Osteosynthesearbeiten zur Festlegung abgesplitteter kleiner Knochenfragmente (B) an einem Hauptknochenteil (A) mit einem glattwandigen Schaftteil (2), an den sich ein einen geringeren Außendurchmesser als das Schaftteil (2) aufweisendes Gewindeteil (3) anschließt, wobei der Übergang vom Gewindeteil (3) zum Schaftteil (2) durch eine sich zum Gewindeteil hin verjüngende konisch gestaltete Stufe (4) gebildet ist, der Gewindeteil (3) mit einem Feingewinde ausgerüstet ist, zumindest das Schaftteil (2) aus einem Werkstoff besteht, der ein Ablängen durch eine Schneidzange ermöglicht und die Größe des Außendurchmessers des Gewindeteiles (3) im wesentlichen 70% - 80 % der Größe des Außendurchmessers des Schaftteiles beträgt.

2. Fixationsstift nach Anspruch 1, dadurch gekennzeichnet, daß das Gewinde des Gewindeteiles (3) als selbstschneidendes Gewinde ausgebildet ist.

3. Fixationsstift nach einem der vorhergehenden Ansprüche, gekennzeichnet durch eine Stützscheibe (5) mit einer Bohrung (6) mit einem Durchmesser größer als der Außendurchmesser des Gewindeteiles (3) und kleiner als der Außendurchmesser des Schaftteiles (2).

4. Fixationsstift nach Anspruch 3, dadurch gekennzeichnet, daß die Form der Bohrung (6) der konisch gestalteten Stufe (4) angepaßt ist.

5. Fixationsstift nach einem der vorhergehenden Ansprüche, dadurch gekennzeichnet, daß die Stützscheibe konkav zum Gewindeteil (3) hin gewölbt ist.

6. Fixationsstift nach einem der vorhergehenden Ansprüche, dadurch gekennzeichnet, daß die äußeren Randkanten (7) der Stützscheibe (5) gewölbt ausgebildet sind.

7. Fixationsstift nach Anspruch 4, dadurch gekennzeichnet, daß die Bohrung (6) mit einem Innengewinde ausgerüstet ist, das mit dem Außengewinde des Gewindeteiles (2) kämmt.

## Claims

1. A fixing pin (1) for osteosynthesis work for the securement of small chipped-off bone fragments (B) to a main bone part (A), having a plain-walled shank part (2) which is adjoined by a threaded part (3) which has a smaller outside diameter than the shank part (2), wherein the transition from the threaded part (3) to the shank part (2) is formed by a step (4) which is fashioned so that it diminishes conically towards the threaded part, the threaded part (3) is equipped with a fine screw thread, at least the shank part (2) consists of a material which enables it to be cut to length by cutting pliers, and the extent of the outside diameter of the threaded part (3) amounts to substantially 70 % - 80 % of the extent of the outside diameter of the shank part.

2. A fixing pin according to claim 1, characterised in that the thread of the threaded part (3) is formed as a self-tapping thread.

3. A fixing pin according to either one of the preceding claims, characterised by a supporting disc (5) having a hole (6) with a diameter larger than the outside diameter of the threaded part (3) and smaller than the outside diameter of the shank part (2).

4. A fixing pin according to claim 3, characterised in that the shape of the hole (6) is matched to the conically fashioned step (4).

5. A fixing pin according to any one of the preceding claims, characterised in that the supporting disc is of concave curvature towards the threaded part (3).

6. A fixing pin according to any one of the preceding claims, characterised in that the external boundary edges (7) of the supporting disc (5) are of curved construction.

7. A fixing pin according to claim 4, characterised in that the hole (6) is equipped with an internal thread which corresponds to the external thread of the threaded part (2).

## Revendications

1. Tige de fixation (1) pour travaux d'ostéosynthèse permettant d'immobiliser de petits fragments osseux détachés (B) sur la partie principale (A) d'un os, cette tige comprenant une partie fût (2) à paroi lisse à laquelle fait suite une partie filetée (3) présentant un plus petit diamètre extérieur que la partie fût (2), la transition entre la partie filetée (3) et la partie fût (2) étant formée par un épaulement (4) qui se rétrécit en cône vers la partie filetée, la partie filetée (3) étant munie d'un filetage fin, la partie fût (2) étant composée d'une matière qui permet de la couper à la bonne longueur au moyen d'une pince coupante, et la valeur du diamètre extérieur de la partie filetée (3) représentant sensiblement 70 %-80 % de la valeur du diamètre extérieur de la partie fût.

2. Tige de fixation selon la revendication 1, caractérisée en ce que le filetage de la partie filetée (3) est constituée par un filetage auto-taraudeur.

3. Tige de fixation selon une des revendications précédentes, caractérisée par une rondelle d'appui (5) présentant un perçage (6) d'un diamètre supérieur au diamètre extérieur de la partie filetée (3) et inférieur au diamètre extérieur de la partie fût (2).

4. Tige de fixation selon la revendication 3, caractérisée en ce que la forme du perçage (6) est adaptée à l'épaulement (4) de configuration conique .

5. Tige de fixation selon une des revendications précédentes, caractérisée en ce que la rondelle d'appui est bombée avec une forme concave en direction de la partie filetée (3).

6. Tige de fixation selon une des revendications précédentes, caractérisée en ce que les arêtes marginales extérieures (7) de la rondelle d'appui (5) sont bombées.

7. Tige de fixation selon la revendication 4, caractérisée en ce que le perçage (6) est muni d'un filetage intérieur qui engrène avec le filetage extérieur de la partie fût (2).
